(19) **Europäisches Patentamt European Patent Office Office européen des brevets**

(11) **EP 4 679 057 A1**

(12) **EUROPEAN PATENT APPLICATION**

(43) Date of publication:
14.01.2026 Bulletin 2026/03

(21) Application number: 25188304.7

(22) Date of filing: 09.07.2025

(51) International Patent Classification (IPC):
*G01N 11/14* (2006.01)   *G01N 33/24* (2006.01)

(52) Cooperative Patent Classification (CPC):
G01N 11/14; G01N 33/24

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 12.07.2024 CA 3248362

(71) Applicant: Soletanche Freyssinet
92500 Rueil Malmaison (FR)

(72) Inventors:
• MCGOWAN, Dallas
EDMONTON, AB T5S 1S (CA)
• VINGBORG, Nicolaj Falker
BURNABY, BC V5A 4W2 (CA)
• WEN JUN ZHENG, Alice
BURNABY, BC V5A 4W2 (CA)
• DOLLING, Ron
BURNABY, BC V5A 4W (CA)
• SUN, Yijia
EDMONTON, T5G 0Y2 (CA)
• OBUSAN, Roque
BURNABY, BC V5A 4W (CA)
• BYRNE, Yasmin
IDEN, TN31 7UT (GB)

(74) Representative: Plasseraud IP
104 Rue de Richelieu
CS92104
75080 Paris Cedex 02 (FR)

(54) **GEOLOGICAL PROPERTIES MEASURING METHOD AND APPARATUS**

(57) A method for measuring mechanical (undrained shear strength, residual shear strength, remolded shear strength, sensitivity, brittleness) and rheological (viscosity, yield stress, thixotropic) properties of soil, the method comprising: providing a measuring apparatus comprising a rotatable vane blade; inserting the vane blade into the soil at a first depth; during a first period of time, accelerating the rotational speed of the vane blade; during a second period of time, decelerating the vane blade while measuring the torque applied to the vane blade and the rotational speed of the vane blade; determining a relationship between the torque and the rotational speed; and determining rheological properties of the soil at the first depth based on the relationship between the torque and the rotational speed.

**FIG. 1**

EP 4 679 057 A1

**Description**

TECHNICAL FIELD

The present invention relates to the technical field of soil and slurry analysis and in particular to a method and an apparatus for measuring both mechanical and rheological properties of soil, sediments, and slurries. In a non-limiting embodiment, the invention relates to the measurements of undrained shear strength, critical shear velocities, plastic viscosity, thixotropy, and/or Bingham yield stress of soils, sediments, tailings or industrial slurries.

BACKGROUND OF THE INVENTION

It is advantageous for many applications to acquire both mechanical and rheological properties of natural or man-made soil, for example for offshore dredging, for preventive measures or emergency planning against landslides.

Another example is the tailing streams produced by mining and mineral processes such as the production of synthetic crude oil. Oil sands tailings are slurries primarily composed of clays, sand, process water, residual bitumen, and a smaller constituent of other organic materials and contaminants. A particularly recalcitrant and clay-dominated subset of oil sands tailings is known as fluid fine tailings (FFT). Due to the dominance of intermolecular forces over microscopic clays FFT remains in stable suspension that requires human intervention prior to reclamation and closure. Oil sands producers have not yet identified a permanent solution to treating FFT and have stored them within the mine leases.

The rheological properties of tailings are important to understand, to achieve effective handling and treatment operations. Parameters such as yield stress and viscosity influence efficient dredge operations, and can be used to model sand entrainment, mixing behavior, and material displacement. Tailings viscosity is also crucial to safety, as it is used to assess run out, the area of impact in the case of a containment breach or failure.

Currently, rheological properties of soils are measured in a laboratory. To measure the viscosity and yield stress of these materials, a rheometer is commonly equipped with a concentric cylinder geometry consisting of a spindle rotating inside a fixed cup. The surface areas of the rotating spindle and fixed cup impart a shear stress onto the fluid material in the narrow anulus of the concentric cylinders. The torque required to maintain an angular velocity of the spindle is used to determine the viscosity of the material.

Issues that arise from the conventional setup stem from the extensive handling and disturbance of the material samples. Sample collection, transportation, and preparation (homogenizing and loading the spindle into the sample and subsequent forcing into the anulus of the concentric cylinder geometry) cause irreversible changes to the shear history of the material. Repeated shearing of slurries treated with polymer reagents will result in permanent mechanical degradation of the flocs, thus returning the material to a near pre flocculated state. Additionally, polymer-amended tailings report artificially low viscosity measurements in the conventional lab setup due to wall slip, where the flocculated fine solids have reduced contact with the frictional surface areas of the cup and spindle.

Additionally, for laboratory testing to be possible, sampling is required, usually from a purpose-built piece of equipment and crew, adding cost and complexity on site. The transportation of the samples implies higher costs, logistics constraints (traceability) and greenhouse gases emissions. Laboratory work is time-consuming, and results are dependent on the chosen methods, personnel expertise, and accumulated shear.

The field vane shear test is a commonly used in situ method to measure the undrained shear strength, and remolded shear strength of soils. The vane is rotated at a standardized, constant rate between 6 and 12 degrees per minute. Vane shearing in clays creates a cylindrical shear plane about the vane and the pore fluid in the soil is unable to escape, therefore a constant volume or undrained failure is achieved. In silty soils which includes many mine tailings, the measurement of shear strengths may be influenced by partial drainage during vane shear testing at standardized, constant rates. To overcome the influence of drainage, a greater rate of shear is selected based on empirical correlation from the pore pressure dissipation test time to 50% dissipation, conducted during a piezocone penetration test (CPTu).

Finally, laboratory testing does not allow for real-time decision making based on immediately obtainable results.

These limitations highlight the need for improved techniques that can overcome these challenges and provide more reliable, cost-efficient and environmentally friendly geotechnical (mechanical strength, drainage effects, sensitivity, brittleness) and/or rheological (thixotropy, viscosity, and yield stress) soil characterization.

SUMMARY OF THE INVENTION

The present disclosure provides for such a need, thanks to a method for measuring properties of soils, tailings, or industrial slurries comprising: providing a measuring apparatus comprising: a vane blade; a variable rate motor configured to rotate the vane blade around an axis; a speed sensor configured to measure a cumulative rotation of the vane blade and a lapsed time and to calculate a rotational speed of the vane blade; and a torque sensor configured to measure a torque exerted on the vane blade; inserting the vane blade into the soil at a first depth; during a first period of time, driving the vane blade in rotation such that the rotational speed of the vane blade increases; during a second period of time, subsequent the first period of time: driving the vane blade in rotation such that the rotational speed of the vane blade decreases; and recording the torque and the rotational speed of the vane blade; determining a relationship between the torque and the rotational speed recorded during the second period of time, at the first depth; and determining properties of the soil at the first depth based on the relationship between the torque and the rotation or rotational speed.

As is explained in further technical detail below, this method reliably performs in-situ measurements of the properties of a soil. The method allows for measurements of geotechnical properties (mechanical strength (undrained shear strength, residual shear strength, remolded shear strength), drainage effects, sensitivity, brittleness) and/or rheological properties (thixotropy, viscosity, and yield stress).

In some examples, determining a relationship between the torque and the rotational speed comprises determining a linear relationship between the torque and the rotational speed.

In some examples, during the first period of time, the rotational speed of the vane blade is increased linearly. The linear increase can be preceded and/or succeeded by a phase of constant rotational speed. The increase and decrease of the rotational speed may be repeated several times to form several cycles, for instance at least 2, at least 3 or at least 5 cycles. Performing several cycles enables to determine the variations of the properties (e.g., the degradation of viscosity) with accumulated shear, also known as thixotropy.

In some examples, during the first period of time, the rotational speed of the vane blade is increased step by step. Here again, several cycles may be performed.

In some examples, during a third period of time, preceding or succeeding the second period of time, driving the vane blade in rotation such that the rotational speed of the vane blade remains constant.

In some examples, the properties of the soil, tailings, or industrial slurries comprise the viscosity of the soil, tailings, or industrial slurries.

In some examples, the properties of the soil, tailings, or industrial slurries comprise the yield stress of the soil, tailings, or industrial slurries.

In some examples, the properties of the soil, tailings, or industrial slurries comprise the thixotropy of the soil, tailings, or industrial slurries.

In some examples, the method further comprises determining at least one of the following properties of the soil, tailings, or industrial slurries: the critical shear velocity; the peak undrained shear strength; the residual undrained shear strength; the remolded undrained shear strength; and the piezometric pressure.

The geotechnical properties are mostly obtained by a relationship between the torque and the rotation (degrees or cumulative angle of rotation) whereas the rheological properties are obtained from a relationship between the torque and the rotational speed. As it is effectively the angle of rotation which is measured, the method and apparatus of the present disclosure may also establish a relationship between the acceleration and the torque.

In some examples, the properties of the soil, tailings, or industrial slurries comprise the brittleness or also known as the sensitivity of the soil, tailings, or industrial slurries.

In some examples, the properties of the soil, tailings, or industrial slurries comprise the critical shear velocity of the soil, tailings, or industrial slurries.

In some examples, the properties of the soil, tailings, or industrial slurries comprise the piezometric pressure of the soil, tailings, or industrial slurries.

In some examples, the method further comprises: inserting the measuring apparatus in the soil, tailings, or industrial slurries at a second depth different from the first depth; and determining properties of the soil, tailings, or industrial slurries at the second depth by: during a third period of time, driving the vane blade in rotation such that the rotational speed of the vane blade increases; during a fourth period of time, subsequent the third period of time: driving the vane blade in rotation such that the rotational speed of the vane blade decreases; and recording the torque and the rotational speed of the vane blade; determining a relationship between the torque and the rotation or rotational speed recorded during the second period of time, at the second depth; and determining properties of the soil, tailings, or industrial slurries at the second depth based on the relationship between the torque and the rotational speed at the second depth. This method can be repeated at several different depths to determine the properties of the soil, tailings, or industrial slurries as a function of the depth.

In some examples, the soil, tailings, or industrial slurries contains at least one of: mine tailings; natural soils; sediments; dredge spoils; or industrial slurries.

The invention also relates to a measuring apparatus for measuring properties of soil, tailings, or industrial slurries comprising: a vane blade; a variable rate motor configured to rotate the vane blade around an axis; a speed sensor configured to measure a rotational speed of the vane blade; and a torque load cell configured to measure a torque exerted on the vane blade.

In some examples, the vane blade comprises a plurality of blades equally distributed around an axle.

In some examples, the plurality of blades comprises four blades.

In some examples, each blade of the plurality of blades comprises two ends and is tapered at one or both of the two ends.

In some examples, each blade of the plurality of blades comprises two ends and is tapered at one of its ends.

In some examples, each blade of the plurality of blades comprises two ends and is not tapered.

In some examples, the vane blade comprises porting for the measurement of piezometric pressure. These pore pressure elements can measure shear induced pore pressures.

In comparison with standard vane testing, combining variable rates, and pore pressure measurements to the vane shear test enables practitioners to validate that undrained conditions were achieved by monitoring the influence of pore pressure versus rotation rate. Furthermore, the ability to accurately control rotation rate across a wide range further enables practitioners to assess the components of drainage, soil fabric, and viscosity.

In some examples, the torque load cell comprises a temperature sensor.

In some examples, the torque load cell comprises at least one inclinometer.

BRIEF DESCRIPTION OF THE DRAWINGS

FIG. 1 shows a top view of a laboratory rheometer.

FIG. 2 shows a top view of a vane blade.

FIG. 3 is a general overview of the measuring apparatus.

FIG. 4 is a graph obtained with the measuring apparatus.

FIG. 5 is diagram of a method for soil characterization.

FIG. 6 is a view of a vane with pore pressure measurement.

FIG. 7 is a schematic graph of the determination of the critical shear velocity.

DETAILED DESCRIPTION OF THE INVENTION

As noted above, the main purpose of the present disclosure is to obtain data relating to the mechanical and rheological properties of the soil.

Geotechnical engineering is a broad branch of civil engineering which involves the design of foundation systems and earthen structures. These designs are dependent on the mechanical properties of soils, broadly termed soil mechanics. The mechanical properties of soils are governed by the minerology of the soil grains, configuration of grains, presence and movement of water, water chemistry, ageing, and other factors. Combining the complexity of the matrix with the vast heterogeneity typical in soils, the field of geotechnical engineering and soil mechanics is complex, requiring substantial efforts to characterize the mechanical properties of soils which govern the soil's ability to take load.

A common tool used in the characterization of the mechanical properties of cohesive soil is the field vane shear test (FVT), which involves inserting a four bladed vane into the soil and rotating the vane. A torque is measured during rotation at constant rotational speed and a cylindrical failure plane in the soil about the vane is assumed. The surface area of this cylinder and the recorded torque are used to determine a shear resistance of the soil versus strain (typically expressed in degrees of rotation). Peak, residual, and remolded shear strengths are interpreted at different levels of strain. The soil sensitivity or brittleness is the ratio of peak to remolded shear strengths. The FVT was originally designed for use in cohesive soils, shearing rates are standardized by the American Society for the Testing of Materials (ASTM) and International Standards Organization (ISO) at rates that are generally accepted to be undrained or constant volume (i.e. no void ratio change) throughout the test in a cohesive or clay dominated soil.

Applying the FVT to silty soils becomes complex due to drainage or partial-drainage during the test at ASTM and ISO prescribed rates of constant shear. Drainage of the shear plane throughout the test results in void ratio change, and soil that can appear to gain strength under shear which is undesirable for geotechnical engineers as it is important to know the lowest bound strength for safe design. A greater rate of rotation is selected to attempt to achieve undrained failure, but currently no technologies exist that allow practitioners to both vary the rate of shear and monitor pore pressure generation throughout the test. Studies by Harvey et al. (Establishing a site-specific standard of practice for field vane shear testing in mine tailings, Proceedings of Tailings and Mine Waste, November 5-8, 2023) have identified the concept of a critical velocity, which is the shear rate at which the lowest bound undrained shear strength in a transitional soil is achieved. Drainage effects increase the shear stress recorded below the critical velocity and viscous effects increase the shear stress recorded at shear rates.

Rheology is a broad branch of physics which involves the flow and deformation of matter. Viscosity refers to a fluid's resistance to flow. For homogenous, single-phase fluids such as liquid water or oil, viscosity tends to be constant at constant temperature. Such fluids are widely referred to as Newtonian fluids. However, slurries and suspensions, especially those with a large constituent of colloidal particles are rarely Newtonian. Aptly named non-Newtonian fluids exhibit complex behaviors such as non-constant viscosity, yield stress, time / shear history dependent properties. There are many rheological models to describe non-Newtonian behavior, but oil sands tailings behavior is commonly represented in literature by the Bingham plastic model. Bingham plastic fluid behavior is characterized by a yield stress which needs to be overcome before flow is initiated, and a plastic viscosity of the fluid once flow begins.

The Bingham plastic fluid model can be summarized by the following equation:

$$\tau = \mu_p\,\dot{\gamma} + \tau_B \tag{1}$$

Where $\tau$ is the shear stress; $\mu_p$ is the plastic viscosity; $\dot{\gamma}$ is the shear rate and $\tau_B$ is the Bingham yield stress. It has to be noted that the Bingham model is only an example and that the present disclosure may appropriately be adapted to use the Herschel-Bulkley model or the Casson model, where rather than using a linear relationship, the relationship respectively involves a polynomial or a square root factor.

Laboratory rheometer instruments employ a concentric cylinder, which features an inner rotating cylindrical spindle within a stationary cup. The annular gap space between spindle and cup contains the fluid being measured, and the dimensions of the spindle and the cup are clearly known. While concentric cylinders are widely used, several shortcomings make this geometry less desirable in industrial slurry applications. First, sample loading into the rheometer involves first partially filling the cup, then inserting the spindle. As the gap between cup and spindle is narrow by design, significant shear is imparted to the fluid sample as it is forced through the gap, which can impact the properties of shear-history dependent fluids irreversibly. Second, for fine-solid laden suspensions, especially those such as oil sands tailings which have also been treated with a polymer-based amendment, wall slip is a critical concern. Wall slip arises from a localized near-wall solids depletion effect, and measurements can be artificially lowered as a result.

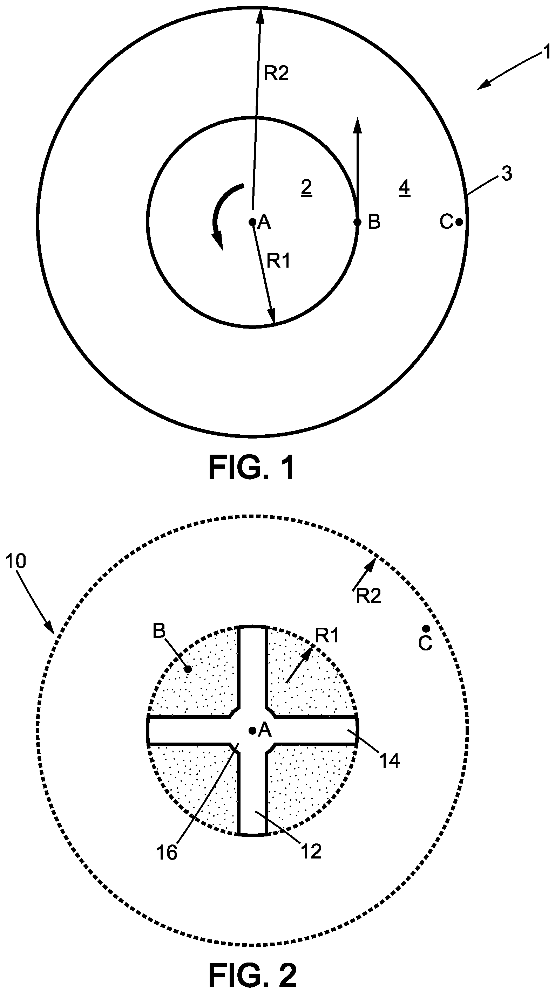

Both of the concerns described can be mitigated or eliminated by using vane blade geometry (see FIG. 2) instead of concentric cylinder (FIG. 1). Due to the slim profile of a vane blade, samples are minimally disturbed during vane blade insertion. While rotating in a fluid exhibiting a yield stress, the fluid encompassed within the blades of the vane can be considered to rotate with the vane as a solid body. Therefore, yielding occurs within the fluid itself, rather than at the boundary between two surfaces, and wall slip is significantly reduced.

Although rheological models such as that described in Equation (1) use shear stress and shear rate, it will be useful for the present disclosure to consider behavior in terms of rotational angular velocity and torque response, which are parameters respectively imposed and measured. The equation describing Bingham plastic fluid behavior in concentric cylinder geometry is (inspired by Shook, C. A., & Roco, M. C. (1991). Slurry Flow: Principles and Practice. Butterworth-Heinemann, ISBN-0750691107; and by Fisher et al. The bucket rheometer for shear stress-shear rate measurement of industrial suspensions, Journal of Rheology, 51, 821, 2007, doi: 10.112211.2750657):

$$\omega = \frac{T}{4\pi L\mu_p}\left(\frac{1}{R_1^2} - \frac{1}{R_2^2}\right) - \frac{\tau_B}{\mu_p}\ln\left(\frac{R_2}{R_1}\right) \tag{2}$$

Where $\omega$ is the angular velocity [rad/s]; T is the torque [N.m]; L is the cylinder length [m]; $\mu_p$ is the Plastic viscosity [Pa.s]; R1 is the Radius of rotating inner cylinder [m]; R2 is the Radius of stationary cup [m]; $\tau_B$ is the Bingham yield stress [Pa].

By re-arranging equation for torque response, we obtain:

$$T = 4\pi L\frac{R_1^2 R_2^2}{R_2^2 - R_1^2}\mu_p\omega + 4\pi L\ln\left(\frac{R_2}{R_1}\right)\frac{R_1^2 R_2^2}{R_2^2 - R_1^2}\tau_B \tag{3}$$

For vane geometry application with yield stress fluids, it is expected that the fluid encompassed within the blades rotates as a solid body cylinder. However, in the case of a vane rotating within a large container of yield stress fluid, the shear at the edge of the rotating surface is always at a maximum, and decays outwards due to frictional losses. In containers which are sufficiently large, such as in a bucket or a tailings pond, the sheared region does not reach the edges of the container. Instead, there exists an area with radius R2 containing fluid which has yielded, beyond which the same fluid essentially behaves as a solid. In this scenario, R2 is not clearly defined, and changes as a function of both fluid properties and the energy imparted by the rotating spindle. Nevertheless, a value was assumed and justified for R2 for the purpose of investigation and proof-of-concept.

In that context, FIG.1 represents a top view of a regular rheometer 1. A cylindrical spindle 2 having a radius R1 is arranged vertically. The cylindrical spindle 2 has a central axis of rotation A. The cylindrical spindle 2 is inserted into a fixed cup 3 having a radius R2. The fixed cup 3 is filled with a medium 4 to be measured. As the cylindrical spindle 2 rotates around axis A, a point noted B adjacent to the cylindrical spindle 2 will be subject to a high shear stress (in the direction of the arrow) whereas a point C close to the fixed cup 3 will substantially see no shear stress. The axial length L of the cylindrical spindle is not visible on this view.

Using Equation (3), one can determine the viscosity and the yield stress of the medium 4 by varying the rotational speed of the cylindrical spindle 2 and simultaneously measuring the torque applied to the spindle (as a result of the resistance of the medium 4).

FIG.2 illustrates the same theory with a rheometer as used as measuring apparatus 10 in the present disclosure. The measuring apparatus 10 comprises a vane blade 12. The vane blade 12 may comprise a plurality of blades 14, for instance 4, distributed around a central shaft 16. The vane blade 12 replaces the cylindrical spindle. The vane blade 12 is driven in rotation around axis A and torque applied to the vane blade 12 is measured. In that case, as there is no fixed cup 3, a theoretical radius R2 is determined, at a location far enough from axis A to not be impacted by any of the shear stress generated by the rotation of the vane blade 12, as explained below. In this figure, point B belongs to a zone of the medium 4 which rotates together with the vane blade 12 and point C is a point which is unimpacted by any shear stress generated from the rotation of the vane blade 12.

Equation (3) reveals that there is a linear relationship between the torque T and the rotational speed $\omega$, i.e. T=slope*$\omega$ + intercept, where:

$$slope = 4\pi L \frac{R_1^2 R_2^2}{R_2^2 - R_1^2} \mu_p \qquad (4)$$

$$intercept = 4\pi L \ln\left(\frac{R_2}{R_1}\right) \frac{R_1^2 R_2^2}{R_2^2 - R_1^2} \tau_B \qquad (5)$$

Since R2 is initially unknown, iterative calculations of the slope and of the intercept with various values of R2 can be determined, for a given couple (L, R1). For example, for L= 300mm and R1=75mm, the slope and the intercept would vary when R2 is below 0.3m, and the slope and the intercept are stable when R2 is at 0.3m or higher. In that case, R2 can be set to be equal to 0.3m. Once R2 is set for a given vane blade geometry (axial length and radius), the assumption is made that R2 is constant throughout an experiment, and thus independent from the velocity of the vane blade (in the tested range) or from the amount of torque received by the vane blade in reaction to its rotation.

An alternative way to determine R2 could be to experiment the rotation of the vane blade near or at the surface of the medium. Optically, one can observe and determine the boundary between moving and stationary fluid. This method is seen as less reliable than the iterative method discussed above, since rotating the vane at the fluid surface is not truly representative of the situation when the vane blade 12 is fully submerged: the energy transfer from a fully submerged vane is expected to be greater than partial submersion.

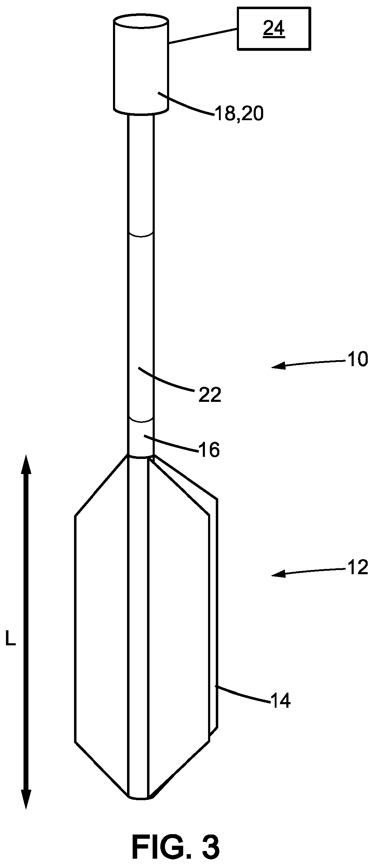

FIG. 3 shows a schematic view of a measuring apparatus 10 which enables to obtain speed and torque data. The measuring apparatus 10 comprises a vane blade 12 comprising a plurality of blades 14 around a shaft 16. The blades 14 may be evenly distributed around the circumference of the shaft 16.

At least part of the shaft 16 and the entirety of the vane blade 12 can be immersed into a medium to be investigated. The shaft 16 can be driven in rotation by a motor 18 around a vertical axis. A speed sensor 20 can measure the instant rotational speed of the motor. The speed sensor 20 can be a shaft encoder. The motor 18 can be a variable rate motor.

The blades 14 may have an outer diameter comprised between 40mm and 150mm.

The design of the shaft 16 may be inspired by cone penetration testing devices. This known technique allows to push down a shaft in the ground. The shaft 16 may be constituted of a series of tubes interconnected together. Each tube may have a diameter comprised between 2cm and 15cm and may have a length comprised between 30cm and 200cm.

The blades 14 have an axial length L which may be comprised between 80mm and 300mm.

A torque sensor 22, for instance a load cell, is arranged to measure the torque applied to the vane blade 12. Indeed, as the vane blade 12 is rotated in a medium, the resistance of the medium to the rotation of the vane blade 12 results in a torque that can be measured, preferably in the vicinity of the vane blade 12. The torque sensor 22 may be arranged at a distance of less than L/5 above the vane blade 12. The torque sensor 22 may be arranged on the shaft 16 and may measure the relative deformation in torsion of the shaft 16, and estimate the torque based on this deformation.

A given torque sensor 22 and/or a given shaft 16 may be used for several sets of blades of different diameters.

The load cell 22 may include a temperature sensor to compensate the strain gauges response based on the measured temperature.

The load cell 22 may include an inclinometer to provide information regarding the verticality of the apparatus.

The motor 18, the speed sensor 20, and the torque sensor 22 may be connected to a control unit 24, which can receive as input, the measurements of the speed sensor 20 and the torque sensor 22 and which can instruct the motor 18 to rotate at a given speed or according to a given speed pattern, as will be explained below. The control unit 24 may comprise any appropriate hardware (processor, memory, communication bus), software or user interface.

The torque and the speed can be continuously monitored by the torque sensor 22 and the speed sensor 20.

For measuring the rheological parameters of a soil, the vane blade 12 is positioned at a given depth, while the motor remains at the surface. The motor 18 drives the shaft 16 in rotation and as the vane blade 12 rotates, the torque and the speed are recorded. Hence, it is possible to establish a relationship between the rotational speed and the torque at a given depth.

The control unit 24 may be programmed to perform the method disclosed above, i.e. controlling the motor 18 to increase the rotational speed of the vane blade 12 at a predetermined acceleration rate for a first period of time, and then decreasing the rotational speed of the vane blade 12 at a predetermined deceleration rate for a second period of time. The control unit 24 receives the speed of the motor from the speed sensor and controls the variable rate motor 18 in a closed loop to very accurately maintain the set rotation rate profile. The control unit 24 may record the torque and the speed continuously in a memory in the second period of time. The control unit 24 may derive a linear relationship between the torque and the speed in the second period of time, and may indicate to a user a viscosity and yield stress calculated from the Equation (4) and (5) above.

The blades 14 may be rectangular or may be tapered as illustrated on FIG. 3. For example at both of their axial ends, the blades 14 may have an edge forming an angle of between 30° and 60°, preferably about 45°, with the vertical axis. In a non-illustrated variant, the blades are tapered only at their bottom edge, or only at their top edge. These various designs may help the vane blade penetrate the medium to investigate or retract into a boring.

The vane geometry coupled with infinite boundary conditions eliminates the slip between the concentric cylinder surfaces and the fluid. Since the fluid encapsulated by the vane blades (point B on FIG. 2) is considered to move with the blades, the measured shear stress is only that of the shearing imparted by the fluid itself.

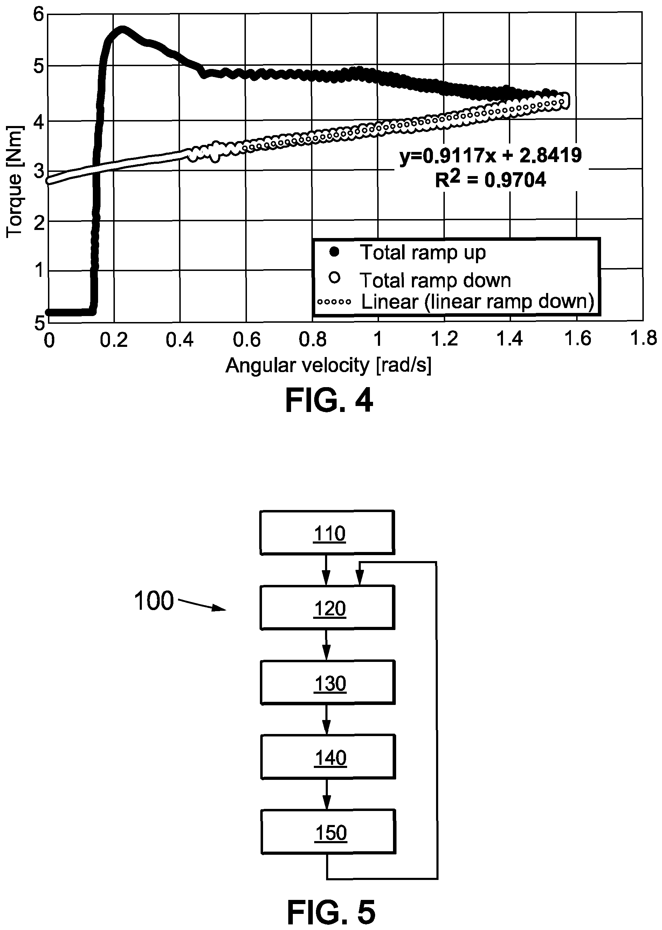

FIG. 4 shows an example of a graph obtained at a given depth. The vane blade 12 is positioned at a given depth. The motor 18 drives the vane blade 12 in rotation from 0 to about 1.6 rad/s over a first period of about 10 minutes. Then, over a second period of about 10 minutes, the motor 18 is driven to decrease the speed of the vane blade 12. The variations of speed may be linear (a ramp up and a ramp down). In a variant, the speed varies step by step.

The second period of time may be directly subsequent the first period of time. Alternatively, a delay may be implemented after the first period of time and before the second period of time to stabilize the mechanical equilibrium at a constant rotational speed. The delay may be a few seconds. The delay may be between 1/20 and 1/10 of the duration of the first period of time.

We can see on FIG. 4 that as the speed decreases, there is a linear relationship between the torque and the speed. As noted in Equations (4) and (5) above, the slope and the intercept are respectively proportional to the plastic viscosity $\mu_p$ and the Bingham yield stress $\tau_B$ (for L, R1 and R2 fixed).

Once the slope and the intercept of the line are calculated, one can determine the viscosity and the yield stress.

The table below gathers some experimental results. Locations A, B and C are geographically distinct locations.

Table 1: experimental results

| Location | Depth | Plastic Viscosity [Pa.s] | Bingham yield stress [Pa] |
|----------|-------|--------------------------|---------------------------|
| A | 2m | 40.31 | 27.19 |
| A | 4m | 79.20 | 41.69 |
| B | 8.3m | 85.19 | 64.93 |
| C | 11.2m | 123.52 | 133.54 |

The calculated values for plastic viscosity falls between 40 Pa.s and 124 Pa.s, roughly comparable to model suspensions of kaolinite and bentonite clay at similar compositions to the measured slurries, and other laboratory testing on true oil sands tailings. The values obtained are representative of the type of tailings and solid content in these samples.

The table below shows additional characterization to assess solid content, density, and clay% by methylene blue index (MBI), as well as clay to water ratio (CWR).

Table 2: experimental results

| Location | Depth | Solid % | Density (kg/L) | MBI (meq/100g) | Clay % | CWR |
|---|---|---|---|---|---|---|
| A | 2m | 48.22% | 1.23 | 8.17 | 58.72 | 0.547 |
| A | 4m | 70.55% | 1.67 | 2.33 | 16.86 | 0.404 |
| B | 8.3m | 47.01% | 1.27 | 6.37 | 45.90 | 0.407 |
| C | 11.2m | 64.64% | 1.41 | 5.23 | 37.76 | 0.595 |

The relationships between sampling location, solid content, density, and rheological parameters follow a logical trend. For example, within the same sampling location A, it makes sense that the 4m deep sample would have a higher solids content, density, and rheological parameters, but lower clay content than the 2m counterpart.

FIG. 5 illustrates a method 100 of the present disclosure. A first step 110 consists in providing a measuring apparatus as described above, i.e. with a vane blade, a variable rate motor configured to rotate the vane blade around an axis, a speed sensor configured to measure a rotational speed of the vane blade, and a torque sensor configured to measure a torque exerted on the vane blade.

In step 120 The vane blade of the measuring apparatus is inserted into the soil at a first depth.

During a first period of time (at step 130), the vane blade is accelerated in rotation.

During a second period of time (at step 140), after the first period of time, the vane blade is decelerated. During that period of time, the torque and the rotational speed of the vane blade are measured.

The acceleration and/or deceleration of the vane blade can be made linearly (ramp up or down) or step by step. Each of these steps may last several minutes, for instance between 3 and 20 minutes, preferably about 10 minutes.

At step 150, based on the measurement of step 140, a relationship between the torque and the rotational speed measured during the second period of time is determined. The relationship may be a linear relationship. Rheological properties of the soil are deduced from this relationship. For example, as described in Equations (4) and (5) above, one can determine the viscosity and the yield stress of the soil at that first depth. Other rheological properties may be deduced from these measurements.

The method can be repeated (as shown with the arrow), where step 120 consists in inserting the vane blade at a second depth, for example deeper than the first depth.

The method may be repeated several times to obtain a vertical cartography of the soil rheological properties at a given geographical location.

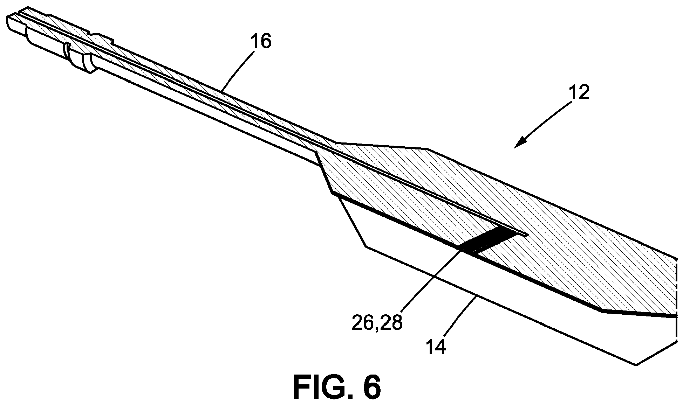

Figure 6 shows a cross-section view of a vane blade 12. In this example, at least one of the blades 12 comprises a series of ports 26 (e.g. from 3 to 10) which receive each a piezoelectric sensor 28. The piezoelectric sensor 28 provides information of pressure at each of the pores. A longitudinal channel 30 is drilled through the shaft 16 for receiving connectors connecting the control unit to the piezoelectric sensors 28.

The ports 26 are arranged at about the center of the blade 12, i.e. axially within a range of 40% and 60% of the total axial length of the blade 12. This is the location where the highest shear-induced pore pressure response is expected.

The ports 26 are arranged at the edge of the blade 12, i.e., facing radially outwardly. This has been found to be the most reliable location and the way to measure the pore pressure which is the less disturbing for the measure of the other properties of the soil.

In summary, the invention is capable of accurately measuring rheological properties of slurries which are challenging to measure with traditional rheometers in a laboratory setting. Measuring in situ eliminates the cost of sampling and laboratory work required by conventional testing. Additionally, as demonstrated above, the presented method allows plausible viscosity readings. The rheological properties of the samples are not altered by retrieval, handling, or preparation for laboratory testing. These in situ experiments aid in the efficiency of dredging, treatment, and pumping operations of tailings materials. Rheological parameters also aid in the modelling of sand entrainment, material displacement, and dam breach run out, allowing for better design of tailings storage facilities.

The invention is differentiated because it allows for the direct measurement of stress, strain rate curves necessary to determine viscosity directly (rather than empirically). Being a direct measure, the in-situ vane viscometer offers a higher level of precision for in-situ measurements than currently available on the market.

The in-situ vane viscometer can also be utilized for conventional geotechnical field vane tests, providing versatility on field investigations where geotechnical and rheological properties need to be understood.

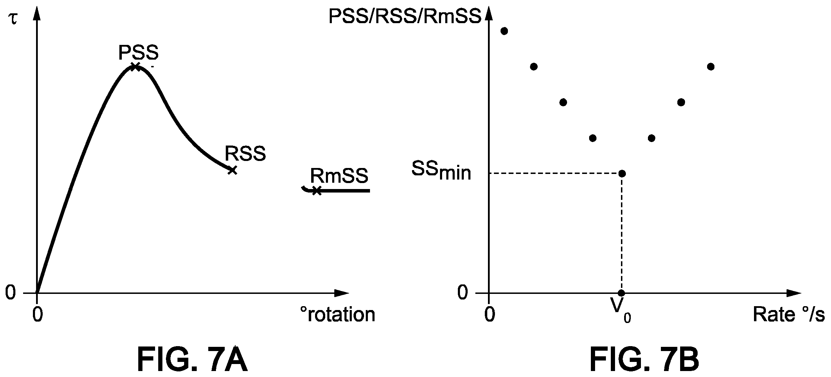

For example, as shown on FIG. 7, the critical shear velocity Vo can be determined. FIG. 7A shows a curve of the shear stress or torque as a function of the cumulative rotation. The torque/shear stress increases up to a peak value PSS and then decreases down to a residual shear strength RSS. This curve can be obtained at a constant rate and over 10 rotations (3600°). A remolded shear strength RmSS can be obtained after that.

Peak, residual, and remolded shear strengths are measured for different rates. The soil sensitivity is the ratio of peak to remolded shear strengths.

Each rate of rotation can be reported on a graph such as FIG. 7B, indicating the shear stress values as a function of the rate used to obtain such a value. The minimum value of all the peaks, here noted SSmin, is obtained for a velocity Vo that is qualified as critical shear velocity. This value can be interpreted as the rate to be used to measure the minimum shear resistance of the soil.

In an example, the above test for obtaining rheological properties as the rotational speed decreases can be integrated into a more complex protocol where other properties such as those shown on FIG. 7. In an example, the vane blade could be run at a constant rate for the first 60 degrees of rotation to obtain the peak shear strength, then undergo a ramping test to get the viscosity and yield stress, ultimately followed by a constant rate of rotation again to get the remolded shear strength.

**Claims**

1.   A method for measuring properties of soil, tailings, or industrial slurries, the method comprising:

   providing a measuring apparatus comprising:

      a vane blade;
      a variable rate motor configured to rotate the vane blade around an axis;
      a speed sensor configured to measure a cumulative rotation of the vane blade and a lapsed time and to calculate a rotational speed of the vane blade; and
      a torque sensor configured to measure a torque exerted on the vane blade;

   inserting the vane blade into the soil at a first depth;
   during a first period of time, driving the vane blade in rotation such that the rotational speed of the vane blade increases;
   during a second period of time, subsequent the first period of time:

      driving the vane blade in rotation such that the rotational speed of the vane blade decreases; and
      recording the torque and the rotational speed of the vane blade;

   determining a relationship between the torque and the rotational speed recorded during the second period of time, at the first depth; and
   determining properties of the soil at the first depth based on the relationship between the torque and the rotational speed.

2.   The method of claim 1, wherein determining a relationship between the torque and the rotational speed comprises determining a linear relationship between the torque and the rotational speed.

3.   The method of claim 1 or 2, wherein during the first period of time, the rotational speed of the vane blade is increased linearly.

4.   The method of any of the preceding claims, wherein during the first period of time, the rotational speed of the vane blade is increased step by step.

5.   The method of any of the preceding claims, further comprising, during a third period of time, preceding or succeeding the second period of time, driving the vane blade in rotation such that the rotational speed of the vane blade remains constant.

6.   The method of any of the preceding claims, wherein the properties of the soil, tailings, or industrial slurries comprise the viscosity of the soil, tailings, or industrial slurries.

7.   The method of any of the preceding claims, wherein the properties of the soil, tailings, or industrial slurries comprise the yield stress of the soil, tailings, or industrial slurries.

8.   The method of any of the preceding claims, wherein the properties of the soil comprise the thixotropy of the soil.

9. The method of any of the preceding claims, further comprising determining at least one of the following properties of the soil, tailings, or industrial slurries: the critical shear velocity; the peak undrained shear strength; the residual undrained shear strength; the remolded undrained shear strength; and the piezometric pressure.

10. The method of any of the preceding claims, further comprising:

inserting the measuring apparatus in the soil, tailings, or industrial slurries at a second depth different from the first depth; and
determining properties of the soil, tailings, or industrial slurries at the second depth by:

during a third period of time, driving the vane blade in rotation such that the rotational speed of the vane blade increases;
during a fourth period of time, subsequent the third period of time:

driving the vane blade in rotation such that the rotational speed of the vane blade decreases; and
recording the torque and the rotational speed of the vane blade;

determining a relationship between the torque and the rotational speed recorded during the second period of time, at the second depth; and
determining properties of the soil, tailings, or industrial slurries at the second depth based on the relationship between the torque and the rotational speed at the second depth.

11. The method of any of the preceding claims, wherein the soil, tailings, or industrial slurries contains at least one of: mine tailings; natural soils, dredge spoils, sediments; and industrial slurries.

12. A measuring apparatus for measuring properties of soil, tailings, or industrial slurries comprising:

a vane blade;
a variable rate motor configured to rotate the vane blade around an axis;
a speed sensor configured to measure a rotational speed of the vane blade; and
a torque load cell configured to measure a torque exerted on the vane blade.

13. The measuring apparatus of claim 12, wherein the vane blade comprises a plurality of blades equally distributed around an axle.

14. The measuring apparatus of claim 12 or 13, wherein each blade of the plurality of blades comprises two ends and is tapered at at least one of the two ends.

15. The measuring apparatus of any of claims 12-14, further comprising a control unit configured to control the variable rate motor to increase the rotational speed of the vane blade during a first period of time and to reduce the rotational speed of the vane blade during a second period of time, subsequent the first period of time.

16. The measuring apparatus of claim 15, wherein the control unit is configured to record the torque and the rotational speed during the second period of time.

17. The measuring apparatus of any of claims 12-16, wherein the torque load cell comprises a temperature sensor.

18. The measuring apparatus of any of claims 12-17, wherein the torque load cell comprises at least one inclinometer.

19. The measuring apparatus of any of claims 12-18, wherein the vane blade comprises porting for the measurement of piezometric pressure.

**FIG. 1**

**FIG. 2**

**FIG. 3**

$$y=0.9117x + 2.8419$$
$$R^2 = 0.9704$$

- ● Total ramp up
- ○ Total ramp down
- ∘∘∘∘∘ Linear (linear ramp down)

FIG. 4

FIG. 5

16

12

26,28

14

**FIG. 6**

**FIG. 7A**

**FIG. 7B**

EP 4 679 057 A1

Europäisches
Patentamt
European
Patent Office
Office européen
des brevets

# EUROPEAN SEARCH REPORT

**Application Number**

EP 25 18 8304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 958 832 A (UNIV BEIJING JIAOTONG) 20 November 2020 (2020-11-20) * the whole document * ----- | 1-19 | INV. G01N11/14 G01N33/24 |
| X | KR 102 205 891 B1 (BIEL INC [KR]) 21 January 2021 (2021-01-21) * paragraph [0084] - paragraph [0140] * * figures 4-6 * ----- | 1-19 | |
| X | KR 102 491 146 B1 (UNIV CHUNG ANG IND ACAD COOP FOUND [KR] ET AL.) 27 January 2023 (2023-01-27) * paragraph [0051] - paragraph [0064] * * figure 2 * ----- | 1-19 | |
| X | US 2014/137638 A1 (LIBERZON ALEXANDER [IL] ET AL) 22 May 2014 (2014-05-22) * paragraph [0224] - paragraph [0234] * * figure 1B * ----- | 12-19 | |

**TECHNICAL FIELDS SEARCHED (IPC)**

G01N

The present search report has been drawn up for all claims

| Place of search | Date of completion of the search | Examiner |
|---|---|---|
| The Hague | 6 November 2025 | Liefrink, Feike |

EPO FORM 1503 03.82 (P04C01)

## ANNEX TO THE EUROPEAN SEARCH REPORT
## ON EUROPEAN PATENT APPLICATION NO.

EP 25 18 8304

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2025

| Patent document cited in search report | | Publication date | Patent family member(s) | | Publication date |
|---|---|---|---|---|---|
| CN 111958832 | A | 20-11-2020 | NONE | | |
| KR 102205891 | B1 | 21-01-2021 | NONE | | |
| KR 102491146 | B1 | 27-01-2023 | NONE | | |
| US 2014137638 | A1 | 22-05-2014 | US 2014137638 A1 | | 22-05-2014 |
| | | | WO 2013001538 A1 | | 03-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARVEY et al.** Establishing a site-specific standard of practice for field vane shear testing in mine tailings. *Proceedings of Tailings and Mine Waste*, 05 November 2023

- **SHOOK, C. A. ; ROCO, M. C.** Slurry Flow: Principles and Practice. Butterworth-Heinemann, 1991
- **FISHER et al.** The bucket rheometer for shear stress-shear rate measurement of industrial suspensions. *Journal of Rheology*, 2007, vol. 51, 821